# EUROPEAN PATENT APPLICATION

(11) **EP 4 822 021 A1**
(43) Date of publication of application: **14.10.2026**
(21) Application number: 24899354.5
(22) Date of filing: 27.08.2024
(51) Int. Cl.: C12N 15/10

(54) **NUCLEIC ACID EXTRACTION METHOD FOR ENGINEERED CELL**

(30) Priority: 06.12.2023 CN 202311656755
(71) Applicant: JW Therapeutics R&D (Shanghai) Co., Ltd., Shanghai 201210 (CN); JW Therapeutics (Shanghai) Co., Ltd., Shanghai 201210 (CN)
(72) Inventor: MA, Chao, Shanghai 201210 (CN)
(74) Representative: Body, Marine
(86) International application number: PCT/CN2024/114780
(87) International publication number: WO 2025/118708

(57) **Abstract**

Provided are a method for extracting nucleic acid in a cell, a reagent kit for extracting nucleic acid in a cell, and a use of a nuclease in the preparation of a pre-treatment reagent for extracting nucleic acid in a cell. The method comprises using a nuclease to treat an engineered cell.

## Description

The present application claims priority to Chinese patent application No. 2023116567552, filed on December 6, 2023. The contents of the above Chinese patent application are incorporated herein by reference in their entireties.

### TECHNICAL FIELD

The present disclosure relates to a method of nucleic acid extraction from engineered cells.

### BACKGROUND

Cell therapy has been used for treating diseases, which involves introducing exogenous nucleic acids into cells using genetic engineering methods, as well as implementing stringent cell testing procedures. However, the introduction of exogenous nucleic acids renders cells more susceptible to contamination, thereby interfering with testing. How to remove contamination while maintaining high testing efficiency remains a technical problem to be solved.

### SUMMARY

The present disclosure provides a method of nucleic acid extraction from engineered cells. The method comprises a pretreatment step prior to the nucleic acid extraction step. The pretreatment step can remove exogenous nucleic acid contamination while maintaining the efficiency of nucleic acid extraction.

In one aspect, the method disclosed herein comprises: a) a pretreatment step comprising a contact step of contacting the engineered cells with a nuclease; and b) a step of extracting intracellular nucleic acids. In one aspect, the present disclosure further provides a pretreatment method of engineered cells, which comprises a pretreatment step comprising a contact step of contacting the engineered cells with a nuclease.

In one aspect, in the method as described above, the nuclease is an endonuclease, an exonuclease, or a combination thereof. In one aspect, the nuclease is a deoxyribonuclease, a ribonuclease, or a universal nuclease. In one aspect, the nuclease is Benzonase or DnaseI.

In one aspect, in the method as described above, the contact lasts for no more than 2 hours. In one aspect, the contact lasts for 15 minutes to 60 minutes. In one aspect, the contact lasts for about 30 minutes. In one aspect, the density of the engineered cells during the contact is 1 × 10⁴ cells/mL to 1 × 10⁸ cells/mL. In one aspect, the density of the engineered cells during the contact is 5 × 10⁵ cells/mL to 1 × 10⁷ cells/mL. In one aspect, the density of the engineered cells during the contact is 5 × 10⁵ cells/mL to 7.5 × 10⁶ cells/mL. In one aspect, the concentration of the nuclease during the contact is 50 to 2000 U/mL. In one aspect, the concentration of the nuclease during the contact is 75 to 1500 U/mL. In one aspect, the concentration of the nuclease during the contact is 100 to 1000 U/mL. In one aspect, the ratio of the nuclease to the cells during the contact is 1 × 10⁴ to 2 × 10⁷ cells per 1000 U of the nuclease. In one aspect, the ratio of the nuclease to the cells during the contact is 5 × 10⁵ to 1.5 × 10⁷ cells per 1000 U of the nuclease. In one aspect, the ratio of the nuclease to the cells during the contact is 1 × 10⁶ to 7.5 × 10⁶ cells per 1000 U of the nuclease.

In one aspect, in the method as described above, the pretreatment step comprises a step of freezing and thawing the engineered cells prior to the contact step. In one aspect, the temperature for freezing the engineered cells is no more than -20°C. In one aspect, the temperature for freezing the engineered cells is no more than -80°C.

In one aspect, the method as described above comprises: a) a pretreatment step comprising contacting the engineered cells with a nuclease for 15 minutes to 60 minutes, wherein the density of the engineered cells during the contact is 5 × 10⁵ cells/mL to 1 × 10⁷ cells/mL, and the concentration of the nuclease is 75 to 1500 U/mL; and b) a step of extracting intracellular nucleic acids. In one aspect, the pretreatment step comprises freezing and thawing the engineered cells, and contacting the thawed engineered cells with a universal nuclease for about 30 minutes, wherein the density of the engineered cells during the contact is 5 × 10⁵ cells/mL to 7.5 × 10⁶ cells/mL, and the concentration of the nuclease is 100 to 1000 U/mL. In one aspect, the pretreatment step comprises freezing and thawing the engineered cells, and contacting the thawed cells with Benzonase for 30 minutes, wherein the density of the engineered cells during the contact is 1 × 10⁶ cells/mL to 7.5 × 10⁶ cells/mL, and the concentration of the nuclease is 1000 U/mL.

In one aspect, the method as described above comprises: a) a pretreatment step comprising a step of freezing and thawing the engineered cells and a contact step of contacting the engineered cells with a nuclease; and b) a step of extracting intracellular nucleic acids. In one aspect, the nuclease is Benzonase. In one aspect, the contact lasts for 30 minutes to 60 minutes. In one aspect, the contact lasts for 30 minutes. In one aspect, the density of the engineered cells during the contact is 5 × 10⁵ cells/mL to 1 × 10⁷ cells/mL. In one aspect, the density of the engineered cells during the contact is 1 × 10⁶ cells/mL to 7.5 × 10⁶ cells/mL. In one aspect, the ratio of the nuclease to the cells during the contact is 1 × 10⁶ to 1 × 10⁷ engineered cells per 1000 U of the nuclease. In one aspect, the nuclease is Benzonase. In one aspect, the pretreatment step comprises freezing and thawing the engineered cells, and contacting the thawed cells with Benzonase for 30 minutes, wherein the density of the engineered cells during the contact is 1 × 10⁶ cells/mL to 7.5 × 10⁶ cells/mL, and the ratio of the nuclease to the cells is 1 × 10⁶ to 1 × 10⁷ engineered cells per 1000 U of the nuclease.

In one aspect, in the method as described above, the engineered cells are engineered mammalian cells. In one aspect, the engineered cells are engineered immune cells or engineered tumor cells. In one aspect, the engineered cells are engineered T cells or engineered NK cells. In one aspect, the engineered cells comprise exogenous nucleic acids introduced via viral vectors, non-viral vectors, or physical methods. In one aspect, the exogenous gene is introduced into the engineered cells via lentiviral vectors, retroviral vectors, adenoviral vectors, adeno-associated viral vectors, cationic liposomes, polymer nanoparticles, transposons, electroporation, or microinjection. In one aspect, the exogenous gene is introduced into the engineered cells via lentiviral vectors.

In another aspect, the present disclosure further provides a kit for nucleic acid extraction from engineered cells, comprising a pretreatment reagent and a nucleic acid extraction reagent, wherein the pretreatment reagent comprises a nuclease. In one aspect, the nuclease is a deoxyribonuclease, a ribonuclease, or a universal nuclease. In one aspect, the nuclease is Benzonase or DnaseI.

In another aspect, the present disclosure further provides the use of a nuclease in the preparation of a pretreatment reagent for nucleic acid extraction from engineered cells. In one aspect, the nuclease is a deoxyribonuclease, a ribonuclease, or a universal nuclease. In one aspect, the nuclease is Benzonase or DnaseI.

### BRIEF DESCRIPTION OF THE DRAWINGS

FIG. 1 shows the effect of cell cryopreservation on nuclease pretreatment as analyzed by agarose gel electrophoresis.
FIG. 2A to FIG. 2D respectively show the DNA molecular weight distribution in Lanes 1 to 4 of the agarose gel electrophoresis.

### DETAILED DESCRIPTION OF THE PREFERRED EMBODIMENT

After reading the present specification, it will become apparent to those skilled in the art how to implement the present disclosure in various alternative embodiments and alternative applications. However, the embodiments described herein are presented by way of illustration only and not by way of limitation. As such, the following detailed description should not be construed as limiting the scope or breadth of the present disclosure as set forth below.

### I. Terminology

The term "a" or "an" may refer to one or more. The term "about" refers to a range within ±5% of the corresponding value. The terms "comprising", "including", and "containing" shall be understood to mean that, in addition to the recited elements, any other feasible elements may also be included, provided that the addition of such elements does not render the technical solution comprising these elements impracticable. The term "consisting of ..." means having only the elements described by "...". The term "comprising" encompasses the situation of "consisting of ...".

The term "engineered cell" refers to a cell containing exogenous nucleic acid or genetic modification.

The term "pretreatment" refers to any pretreatment capable of improving the efficiency of intracellular nucleic acid extraction. For example, in the present disclosure, "pretreatment" involves contacting cells with nucleases, surfactants, or the like. Pretreatment does not or substantially does not cause cell lysis.

The term "nuclease" refers to an enzyme capable of catalyzing the hydrolysis of bonds between nucleic acids within a DNA or RNA molecule.

The term "contact" refers to the interaction, reaction, or physical contact of two or more substances. For example, in the present disclosure, "contact" refers to the process of mixing engineered cells with nucleases. During the contact, the concentration or density of a substance refers to the concentration or density of the substance in a contact medium (*e.g.*, a mixture).

The term "immune cell" refers to a cell involved in an immune response designed to protect an organism from foreign substances, viruses, and cells. Immune cells may be derived from many organs and tissues, such as the thymus, spleen, lymph nodes, lymphoid tissue clusters (*e.g.*, in the gastrointestinal tract and bone marrow). Such cells include T cells, B cells, natural killer cells, macrophages, neutrophils, tumor-infiltrating lymphocytes, dendritic cells, mast cells, eosinophils, and basophils, as well as progenitor cells that develop into these cells. In the present disclosure, immune cells are modified (*e.g.*, by introduction of exogenous nucleic acids) prior to nucleic acid extraction to become engineered cells.

### II. Cell treatment methods

The present disclosure provides a method of intracellular nucleic acid extraction, comprising a pretreatment step and an extraction step. The present disclosure further provides a pretreatment method of engineered cells, comprising a pretreatment step. In one aspect, the intracellular nucleic acid is intracellular DNA. In one aspect, the intracellular nucleic acid is genomic DNA of a cell. Surprisingly, the method disclosed herein can significantly reduce contamination of intracellular nucleic acids by impurities while maintaining a high recovery rate, compared to a method of intracellular nucleic acid extraction lacking a pretreatment step.

### 2.1. Pretreatment step

In one aspect, the pretreatment step comprises a contact step of contacting the engineered cells with a nuclease. In one aspect, the nuclease is an endonuclease, an exonuclease, or a combination thereof. In one aspect, the nuclease is a deoxyribonuclease, a ribonuclease, or a universal nuclease. The universal nuclease exhibits DNase and RNase activity. In one aspect, the nuclease exhibits no or substantially no activity for degrading proteins or disrupting cell membrane structures. In one aspect, the nuclease is Benzonase or DnaseI. In one aspect, the nuclease is Benzonase.

In one aspect, the contact lasts for no more than 6 hours, for example, no more than 1, 2, 3, 4, 5, or 6 hours. In one aspect, the contact lasts for 1 to 60 minutes, for example, 1, 5, 15, 20, 25, 30, 35, 40, 45, 50, 55, or 60 minutes. In one aspect, the contact lasts for 5 minutes to 60 minutes. In one aspect, the contact lasts for 15 minutes to 60 minutes. In one aspect, the contact lasts for 20 minutes to 40 minutes. In one aspect, the contact lasts for about 30 minutes or 30 minutes. In one aspect, the contact is performed at 25°C to 42°C. In one aspect, the contact is performed at 25°C, 37°C, or 42°C. In one aspect, the contact is performed at about 37°C or 37°C.

In one aspect, the density of the engineered cells during the contact is 1 × 10⁴ cells/mL to 1 × 10⁸ cells/mL. In one aspect, the density of the engineered cells during the contact is 1 × 10⁴ cells/mL, 5 × 10⁴ cells/mL, 1 × 10⁵ cells/mL, 5 × 10⁵ cells/mL, 1 × 10⁶ cells/mL, 5 × 10⁶ cells/mL, 7.5 × 10⁶ cells/mL, 1 × 10⁷ cells/mL, 5 × 10⁷ cells/mL, or 1 × 10⁸ cells/mL. In one aspect, the density of the engineered cells during the contact is 5 × 10⁴ cells/mL to 1 × 10⁷ cells/mL. In one aspect, the density of the engineered cells during the contact is 1 × 10⁵ cells/mL to 7.5 × 10⁶ cells/mL. In one aspect, the density of the engineered cells during the contact is 5 × 10⁵ cells/mL to 7.5 × 10⁶ cells/mL. In one aspect, the density of the engineered cells during the contact is about 5 × 10⁵ cells/mL, 1 × 10⁶ cells/mL, 5 × 10⁶ cells/mL, or 7.5 × 10⁶ cells/mL.

In one aspect, the concentration of the nuclease during the contact is 10 to 5000 U/mL. In one aspect, the concentration of the nuclease during the contact is 50 to 2000 U/mL. In one aspect, the concentration of the nuclease during the contact is 50 to 1500 U/mL. In one aspect, the concentration of the nuclease during the contact is 100 to 1500 U/mL. In one aspect, the concentration of the nuclease during the contact is 50 U/mL, 75 U/mL, 100 U/mL, 200 U/mL, 300 U/mL, 400 U/mL, 500 U/mL, 600 U/mL, 700 U/mL, 800 U/mL, 900 U/mL, 1000 U/mL, 1500 U/mL, 2000 U/mL, 3000 U/mL, 4000 U/mL, or 5000 U/mL. In one aspect, the concentration of the nuclease during the contact is 100 to 1000 U/mL. In one aspect, the concentration of the nuclease during the contact is about 100, 500, or 1000 U/mL. In one aspect, the concentration of the nuclease during the contact is about 1000 U/mL.

In one aspect, the ratio of the nuclease to the cells during the contact is 1 × 10⁴ to 1 × 10⁸ engineered cells per 1000 U of the nuclease. In one aspect, the ratio of the nuclease to the cells during the contact is 1 × 10⁴ to 2 × 10⁷ engineered cells per 1000 U of the nuclease. In one aspect, the ratio of the nuclease to the cells during the contact is 1 × 10⁴, 5 × 10⁴, 1 × 10⁵, 5 × 10⁵, 1 × 10⁶, 5 × 10⁶, 7.5 × 10⁶, 1 × 10⁷, 2 × 10⁷, 5 × 10⁷, or 1 × 10⁸ engineered cells per 1000 U of the nuclease. In one aspect, the ratio of the nuclease to the cells during the contact is 5 × 10⁵ to 1.5 × 10⁷ engineered cells per 1000 U of the nuclease. In one aspect, the ratio of the nuclease to the cells during the contact is 5 × 10⁵ to 1 × 10⁷ engineered cells per 1000 U of the nuclease. In one aspect, the ratio of the nuclease to the cells during the contact is 1 × 10⁶ to 7.5 × 10⁶ engineered cells per 1000 U of the nuclease. In one aspect, the concentration of the nuclease during the contact is 100 U/mL and the cell density is 5 × 10⁵ cells/mL. In one aspect, the concentration of the nuclease during the contact is 500 U/mL and the cell density is 5 × 10⁶ cells/mL. In one aspect, the concentration of the nuclease during the contact is 1000 U/mL and the cell density is 1 × 10⁶ cells/mL. In one aspect, the concentration of the nuclease during the contact is 1000 U/mL and the cell density is 7.5 × 10⁶ cells/mL. In one aspect, the concentration of the nuclease during the contact is 1000 U/mL and the cell density is 1 × 10⁷ cells/mL.

In one aspect, the contact is performed in a liquid medium. In one aspect, the contact is performed in a liquid medium suitable for cell survival. In one aspect, the contact is performed in a buffer suitable for cell survival. In one aspect, the contact is performed in phosphate-buffered saline. In one aspect, the contact is performed in Dulbecco's phosphate-buffered saline (DPBS). In one aspect, the buffer contains 1 to 10 mM Mg²⁺. In one aspect, the buffer contains 1, 2, 3, 4, 5, 6, 7, 8, 9, or 10 mM Mg²⁺. In one aspect, the buffer contains 1, 2, 3, 4, 5, 6, 7, 8, 9, or 10 mM Mg²⁺. In one aspect, the buffer contains 1 to 5 mM Mg²⁺. In one aspect, the buffer contains 1 to 3 mM Mg²⁺. In one aspect, the buffer contains about 2 mM or 2 mM Mg²⁺.

In one aspect, the method comprises a step of freezing and thawing the engineered cells prior to the contact step. In one aspect, the freezing is performed under conditions suitable for cell cryopreservation. In one aspect, the temperature for freezing is no more than -20°C, -30°C, -40°C, -50°C, -60°C, -70°C, -80°C, -90°C, or -100°C. In one aspect, the temperature for freezing is -20°C to -100°C. In one aspect, the temperature for freezing is - 60°C to -80°C. In one aspect, the temperature for freezing is about -80°C or -80°C. In one aspect, the freezing is performed under liquid nitrogen conditions. In one aspect, the freezing lasts for 1, 2, 3, 6, 12, 24, 48, or 72 hours, or 5 days, 7 days, or 14 days. In one aspect, the freezing lasts for 6 hours to 7 days. In one aspect, the freezing lasts for 24 hours to 3 days. In one aspect, the thawing is performed at 0°C to 37°C. In one aspect, the thawing is performed at room temperature, about 25°C, or 25°C. In one aspect, the engineered cells are present in a cryoprotectant solution. In one aspect, the cryoprotectant solution may be formulated in any manner known in the art. In one aspect, the cryoprotectant solution is DMEM culture medium. In one aspect, the cryoprotectant solution comprises 0 to 20% DMSO, 0 to 40% serum, and 50 to 100% DMEM culture medium. In one aspect, the cryoprotectant solution comprises 5 to 10% DMSO, 10 to 20% serum, and 70 to 85% DMEM culture medium. In one aspect, the cryoprotectant solution comprises 10% DMSO, 20% fetal bovine serum, and 70% DMEM culture medium.

In one aspect, the pretreatment step further comprises a washing step following the contact step. In one aspect, the washing step comprises washing the cells by centrifugation with a buffer. In one aspect, the buffer is any buffer suitable for cell washing known in the art. In one aspect, the buffer is PBS or DPBS. In one aspect, the volume of buffer used for a single wash is 0.5, 1, 2, 3, 4, 5, 6, 7, 8, 9, or 10 mL. In one aspect, the cells are washed 1, 2, 3, 4, 5, 6, 7, 8, 9, or 10 times. In one aspect, the cells are washed twice by centrifugation with 1 mL of DPBS.

### 2.2. Extraction step

In one aspect, the extraction step may employ various techniques known to those skilled in the art. An exemplary extraction method comprises: (i) disrupting cell membranes (*i.e.*, cell lysis), and (ii) extracting intracellular nucleic acids. Methods of cell lysis are well known to those skilled in the art. For example, various mechanical shearing or ultrasonic techniques may be used for disrupting cell membranes. The cell lysis step further involves the use of detergents and surfactants to dissolve lipids in cell membranes and nuclear membranes. In one aspect, the lysis step may further involve the use of proteases to break down proteins and/or the use of RNases to digest RNA in the sample. Methods of nucleic acid extraction are also well known. For example, extraction may be performed using organic solvents (*e.g.*, a mixture of phenol and chloroform), followed by precipitation with ethanol. Other suitable methods include salting-out DNA extraction, cetyltrimethylammonium bromide DNA extraction, and guanidinium thiocyanate DNA extraction. A variety of kits are commercially available for extracting DNA from biological samples, such as the QIAamp kit from Qiagen (Germantown, MD), or the Maxwell^{®} and ReliaPrep^{™} kits from Promega (Madison, WI).

### III. Kits

In one aspect, the present disclosure provides a kit for intracellular nucleic acid extraction, comprising a pretreatment reagent and an intracellular nucleic acid extraction reagent. In another aspect, the present disclosure provides the use of a nuclease in the preparation of a pretreatment reagent for intracellular nucleic acid extraction. In one aspect, the nuclease is a deoxyribonuclease, a ribonuclease, or a universal nuclease. The universal nuclease exhibits DNase and RNase activity. In one aspect, the nuclease exhibits no or substantially no activity for degrading proteins or disrupting cell membrane structures.

In one aspect, the concentration of the nuclease in the pretreatment reagent is 10 to 5000 U/mL. In one aspect, the concentration of the nuclease in the pretreatment reagent is 50 to 2000 U/mL. In one aspect, the concentration of the nuclease in the pretreatment reagent is 50 to 1500 U/mL. In one aspect, the concentration of the nuclease in the pretreatment reagent is 100 to 1500 U/mL. In one aspect, the concentration of the nuclease in the pretreatment reagent is 50 U/mL, 75 U/mL, 100 U/mL, 200 U/mL, 300 U/mL, 400 U/mL, 500 U/mL, 600 U/mL, 700 U/mL, 800 U/mL, 900 U/mL, 1000 U/mL, 1500 U/mL, 2000 U/mL, 3000 U/mL, 4000 U/mL, or 5000 U/mL. In one aspect, the concentration of the nuclease in the pretreatment reagent is 100 to 1000 U/mL. In one aspect, the concentration of the nuclease is about 100, 500, or 1000 U/mL. In one aspect, the concentration of the nuclease is about 1000 U/mL.

In one aspect, the kit comprises an intracellular nucleic acid extraction reagent. Such nucleic acid extraction reagents are well known to those skilled in the art, including, for example, detergents capable of lysing cells, surfactants capable of dissolving lipids in cell membranes and nuclear membranes, proteases, RNases, and organic solvents for nucleic acid extraction (*e.g.*, a mixture of phenol and chloroform), salts, cetyltrimethylammonium bromide, and guanidinium thiocyanate. In one aspect, the kit comprises commercially available reagents, such as reagents from the QIAamp kit from Qiagen or the Maxwell^{®} and ReliaPrep^{™} kits from Promega. In one aspect, the pretreatment reagents and intracellular nucleic acid extraction reagents in the kit are packaged together or separately. A kit comprising only pretreatment reagents, while describing intracellular nucleic acid extraction reagents in the instructions, also falls within the scope of protection of the present disclosure.

### IV. Cells

In one aspect, the cells involved in the method disclosed herein are engineered to contain exogenous genes. In one aspect, the cells are mammalian cells. In one aspect, the cells are human cells. In one aspect, the cells are immune cells or enriched immune cells. In one aspect, the cells are tumor cells. In one aspect, the cells are T cells or enriched T cells. In one aspect, the cells are CD4⁺ T cells or enriched CD4⁺ T cells. In one aspect, the cells are CD8⁺ T cells or enriched CD8⁺ T cells. In one aspect, the cells are CD4⁺ T cells and CD8⁺ T cells. In one aspect, the cells are enriched CD4⁺ T cells and enriched CD8⁺ T cells. In one aspect, the cells comprise engineered cells or an enriched population of engineered cells. In one aspect, the cells comprise genetically engineered T cells or an enriched population of genetically engineered T cells. In one aspect, the cells comprise chimeric antigen receptor (CAR)-expressing T cells or an enriched population of CAR-expressing T cells. In one aspect, the cells have undergone a freeze-thaw cycle.

In one aspect, a preparation method for the engineered cells comprises cell isolation, selection, activation, transduction, cultivation, expansion, washing, suspension, dilution, concentration, and/or formulation. In one aspect, the method comprises isolating cells, and preparing, processing, and culturing the cells under stimulating conditions. In one aspect, the method comprises isolating cells from a biological sample, and incubating the isolated cells with a viral vector to transduce an exogenous nucleic acid into the cells. In one aspect, the method comprises isolating cells from a biological sample, incubating the isolated cells with a viral vector to transduce an exogenous nucleic acid into the cells, and culturing the transduced cells. In one aspect, the isolation comprises a step of selecting cells. In one aspect, the transduction is performed after stimulating the isolated cells with a stimulation reagent.

In one aspect, the preparation method comprises one or more of the following: (a) pre-washing a biological sample containing cells (*e.g.*, a whole blood sample, buffy coat sample, peripheral blood mononuclear cell (PBMC) sample, unfractionated T cell sample, lymphocyte sample, leukocyte sample, apheresis product, or leukapheresis product); (b) isolating (*e.g.*, selecting) desired cells (*e.g.*, CD4⁺ and/or CD8⁺ T cells) from the sample, for example, by incubating the cells with an immunoaffinity reagent; (c) introducing a vector encoding an exogenous nucleic acid (*e.g.*, a recombinant receptor) into the isolated or selected cells, for example, by incubating the isolated (*e.g.*, selected) cells with a viral vector encoding the recombinant receptor; and (d) culturing or expanding the cells. In one aspect, the method may further comprise a step of activating the cells by exposing the cells to stimulating conditions, which may be performed before, during, and/or after incubation of the cells with the viral vector, for example, between steps (b) and (c). In one aspect, the viral vector is a lentiviral vector. In one aspect, the washing and/or suspension steps may also be performed before or after any of the aforementioned steps. In one aspect, one or more, or all of the steps of the cell culture method are performed under sterile conditions. In some embodiments of such methods, cell isolation, transduction, washing, optionally activation or stimulation, and formulation are all performed within a closed system.

In one aspect, the cell culture method comprises a genetic engineering step. In one aspect, the step involves introducing an exogenous nucleic acid into the cells. In one aspect, the genetic engineering step comprises introducing a recombinant protein into the cells via a vector. Such vectors include viral and non-viral systems. In one aspect, the viral system comprises recombinant infectious viral particles, such as adenoviral vectors, adeno-associated viral (AAV) vectors, human immunodeficiency viral (HIV) vectors, recombinant lentiviral vectors, or retroviral vectors (*e.g.*, gamma-retroviral vectors). In one aspect, the non-viral system comprises a transposon system, such as the PiggyBac or Sleeping Beauty gene transfer system. In one aspect, the step is performed by electroporation. In one aspect, the step is performed by transduction, transposons, electroporation, or a combination thereof. Other methods of introducing and expressing genetic material in immune cells include calcium phosphate transfection, protoplast fusion, cationic liposome-mediated transfection, tungsten particle-facilitated microprojectile bombardment, and strontium phosphate DNA coprecipitation.

### V. Exogenous nucleic acids

In one aspect, the cells involved in the method disclosed herein comprise an exogenous nucleic acid encoding a recombinant protein. In one aspect, the recombinant protein is a chimeric receptor, a chimeric antigen receptor (CAR), a T cell receptor (TCR), a chimeric antibody-T cell receptor construct (caTCR), a chimeric signaling receptor (CSR), or a combination thereof.

In one aspect, the recombinant protein is a chimeric antigen receptor (CAR). In one aspect, the antigen is selectively expressed or overexpressed on cells associated with a disease or disorder (*e.g.*, tumor cells or pathogenic cells) compared to normal or non-targeted cells. In one aspect, the diseases and disorders include proliferative, neoplastic, and malignant diseases and disorders, including cancers and tumors, including hematological cancers and cancers of the immune system, such as lymphomas, leukemias, and/or myelomas, such as B-cell leukemia, T-cell leukemia, myeloid leukemia, lymphoma, and multiple myeloma. In one aspect, the CAR comprises an extracellular antigen recognition domain that specifically binds to an antigen. Accordingly, the extracellular antigen recognition domain that specifically binds to an antigen comprises one or more antigen-binding molecules, such as one or more antigen-binding fragments, domains, or moieties, or one or more antibody variable domains, and/or antibodies. In one aspect, the antigen-binding molecule is a full-length antibody, such as a single-domain antibody, a monospecific antibody, or a multispecific antibody; or an antibody fragment, such as an scFv. In one aspect, the CAR comprises a spacer, which may be or comprise at least a portion of an immunoglobulin constant region or a variant or modified form thereof, such as a hinge region and/or a CH1/CL region and/or an Fc region. In one aspect, the spacer is located between the extracellular antigen recognition domain and the transmembrane domain. In one aspect, the CAR comprises a transmembrane domain. In one aspect, the transmembrane domain is derived from a natural source or a synthetic source. In the case of the natural source, the transmembrane domain is derived from any membrane-bound protein or transmembrane protein. The transmembrane domain comprises one or more of the following: the α, β, or ζ chain of a T cell receptor, CD28, CD3ε, CD45, CD4, CD5, CD8, CD9, CD16, CD22, CD33, CD37, CD64, CD80, CD86, CD134, CD137, and CD154. In one aspect, the transmembrane domain is a synthetic transmembrane domain. In some aspects, the synthetic transmembrane domain primarily comprises hydrophobic amino acid residues, such as leucine and valine. In one aspect, the CAR comprises an intracellular signaling domain, wherein the intracellular signaling domain comprises a cytoplasmic signaling domain, such as an intracellular domain capable of inducing a primary activation signal in T cells, for example, the ζ chain of CD3ζ; and/or the intracellular signaling domain comprises an immunoreceptor tyrosine-based activation motif (ITAM). In one aspect, the CAR comprises a co-stimulatory domain. In one aspect, the co-stimulatory domain is a signaling region and/or transmembrane portion of CD28, 4-1BB, OX40, DAP10, or ICOS. In some aspects, the same CAR comprises both a primary activation signaling region and a co-stimulatory component. In one aspect, the same CAR comprises both a primary activation signaling domain and a co-stimulatory domain.

### VI. Examples

After reading the present specification, it will become apparent to those skilled in the art how to implement the present disclosure in various alternative embodiments and alternative applications. However, the embodiments described herein are presented by way of illustration only and not by way of limitation. As such, the following detailed description should not be construed as limiting the scope or breadth of the present disclosure as set forth below.

In the following examples, unless otherwise specified, the cryopreservation solution comprises 10% DMSO, 20% fetal bovine serum, and 70% DMEM culture medium. DNA extraction was performed as follows: lysing cells followed by treatment with proteinase K to remove proteins bound to DNA; adsorbing free DNA using silica columns or magnetic beads; washing the DNA with an ethanol solution to remove impurities; and eluting the DNA adsorbed on the silica columns or magnetic beads using a low-salt solution. Gene copy numbers were analyzed using techniques well known in the art or commercially available kits.

### Example 1

In this example, the effects of different treatment methods on DNA extraction efficiency and cell analysis were described. The cells used in this example were engineered T cells. Prior to analysis, exogenous nucleic acids were introduced into the cells using a lentiviral vector. The cells were dispersed in cryopreservation solution at a density of 1 × 10⁷ to 2 × 10⁷ cells/mL and stored at -80°C or in liquid nitrogen for at least 12 hours. The cryopreserved cells were thawed at room temperature and collected by centrifugation. A nuclease treatment solution was prepared using DPBS containing 100 U/mL Benzonase and 2 mM Mg²⁺. The cells were treated using the methods described in Groups 1 to 6 below.

**Table 1: Cell treatment methods**

| Group | Treatment method |
|---|---|
| 1 | 5 × 10⁵ cells were washed twice by centrifugation with 1 mL of DPBS |
| 2 | 5 × 10⁵ cells were incubated in 1 mL of nuclease treatment solution for 30 minutes and washed twice by centrifugation with 1 mL of DPBS |
| 3 | 5 × 10⁵ cells were incubated in 1 mL of nuclease treatment solution for 60 minutes and washed twice by centrifugation with 1 mL of DPBS |
| 4 | 5 × 10⁵ cells were washed twice by centrifugation with 1 mL of 0.1% Tween 20 |
| 5 | 5 × 10⁵ cells were washed twice by centrifugation with 1 mL of 0.05% Tween 20 |
| 6 | 5 × 10⁵ cells were washed four times by centrifugation with 1 mL of DPBS |

DNA was extracted and DNA concentration was quantified using a UV spectrophotometer. The relative recovery rate for each treatment method was calculated using the DNA concentration of Group 1 as a reference. VSV-G (vesicular stomatitis virus glycoprotein G) can adsorb onto the cell membrane during lentiviral transduction. The ACTB (human β-actin) gene is present in the cellular genome. DNA samples at 20 ng/µL were subjected to quantitative real-time PCR to evaluate the effects of different treatment methods on the analysis of intranuclear DNA and extracellular adsorbed DNA. The relative impurity removal rate for each treatment method was calculated using the VSV-G content of Group 1 as a reference. The results are shown in Table 2.

**Table 2: Cell treatment efficiency**

| Group | DNA concentration (ng/µL) | Relative recovery rate | VSV-G (copies) | Relative impurity removal rate | ACTB (Ct) |
|---|---|---|---|---|---|
| 1 | 54.43 | N/A | 136.62 | N/A | 19.39 |
| 2 | 54.43 | 98.35% | 39.67 | 70.96% | 19.77 |
| 3 | 46.53 | 85.49% | 37.98 | 72.20% | 19.48 |
| 4 | 58 | 106.55% | 156.82 | -14.79% | 19.40 |
| 5 | 67.67 | 124.31% | 159.72 | -16.91% | 19.25 |
| 6 | 28.13 | 51.68% | 106.69 | 21.90% | 19.78 |

The results demonstrated that, compared to conventional washing with surfactants and buffers, nuclease treatment was more effective in removing impurities adsorbed on the surface. Surprisingly, nuclease treatment for 30 minutes not only effectively removed impurities but also maintained a recovery rate comparable to that of the group without nuclease treatment. Moreover, none of the treatment methods resulted in changes in the Ct value of the ACTB gene, indicating that the genomic DNA of the cells in each group was not degraded.

### Example 2

In this example, the effects of different testing conditions on cell analysis were evaluated. The cells used in this example were human T cells. Prior to analysis, exogenous nucleic acids were introduced into the cells using a lentiviral vector. The cells were dispersed in cryopreservation solution at a density of 1 × 10⁷ to 2 × 10⁷ cells/mL and stored at -80°C or in liquid nitrogen for at least 12 hours. The cryopreserved cells were thawed at room temperature and collected by centrifugation. According to Table 3, DPBS containing 2 mM Mg²⁺ was used as a blank treatment solution (Group 1), and Benzonase was added to the blank treatment solution to prepare nuclease-containing treatment solutions (Groups 2 to 5). The cells were incubated in 1 mL of treatment solution for 30 minutes and washed twice by centrifugation with 1 mL of DPBS, followed by DNA extraction. The results demonstrated that the treatment solutions of the present disclosure are suitable for samples varying in cell density. Under the same cell-to-enzyme ratio, reducing the cell density can improve the clearance of residual plasmids.

**Table 3: Effect of nuclease treatment on gene stability**

| Group | Treatment solution formulation | Number of cells | Residual plasmid (copies/µL) | Clearance (%) |
|---|---|---|---|---|
| 1 | Blank treatment solution | 5 × 10⁶ | 1819.52 | |
| 2 | Treatment solution containing 500 U/mL nuclease | 5 × 10⁶ | 422.65 | 76.77 |
| 3 | Treatment solution containing 1000 U/mL nuclease | 1 × 10⁶ | 336.26 | 81.52 |
| 4 | Treatment solution containing 1000 U/mL nuclease | 7.5 × 10⁶ | 458.08 | 74.82 |
| 5 | Treatment solution containing 1000 U/mL nuclease | 1 × 10⁷ | 750.19 | 58.77 |

### Example 3

In this example, the effect of cell cryopreservation on cell analysis was evaluated. The cells used in this example were human T cells. Prior to analysis, exogenous nucleic acids were introduced into the cells using a lentiviral vector. The transduced cells were divided into two portions. Some cells were dispersed in culture medium at a density of 1 × 10⁵ to 1 × 10⁷ cells/mL, followed by centrifugation. The rest of the cells were dispersed in cryopreservation solution at a density of 1 × 10⁵ to 1 × 10⁷ cells/mL and stored at -80°C for at least 12 hours. The cryopreserved cells were thawed at room temperature, followed by centrifugation. A nuclease treatment solution was prepared using DPBS containing 1000 U/mL nuclease and 2 mM Mg²⁺. The pellets of non-cryopreserved cells or cryopreserved cells were incubated in 1 mL of nuclease treatment solution at 37°C for 30 minutes. The cells were washed twice by centrifugation with 1 mL of DPBS, followed by DNA extraction.

ALB (albumin, human serum albumin) was quantified using qPCR. The results are shown in Table 4. The cryopreserved cells exhibited greater resistance to nuclease treatment, with higher genomic DNA stability compared to the non-cryopreserved cells.

**Table 4: Effect of cryopreservation on gene stability after nuclease treatment**

| Sample No. | ALB (copies) extracted without cryopreservation | ALB (copies) extracted after cryopreservation |
|---|---|---|
| Sample 1 | 15989.56 | 42549.32 |
| Sample 2 | 7124.07 | 40507.96 |
| Sample 3 | 19732.58 | 52532.61 |
| Sample 4 | 12370.67 | 49484.14 |
| Sample 5 | 16169.28 | 50425.62 |

### Example 4

In this example, the effect of cell cryopreservation on cell analysis was evaluated. The cells used in this example were HT1080 cells. Prior to analysis, exogenous nucleic acids were introduced into the cells using a lentiviral vector. The transduced cells were divided into two portions. Some cells were dispersed in culture medium at a density of 1 × 10⁵ to 1 × 10⁷ cells/mL, followed by centrifugation. The rest of the cells were dispersed in cryopreservation solution at a density of 1 × 10⁵ to 1 × 10⁷ cells/mL and stored at -80°C for at least 12 hours. The cryopreserved cells were thawed at room temperature, followed by centrifugation. A nuclease treatment solution was prepared using DPBS containing 1000 U/mL nuclease and 2 mM Mg²⁺. The pellets of non-cryopreserved cells or cryopreserved cells were incubated in 1 mL of nuclease treatment solution at 37°C for 30 minutes. The cells were washed twice by centrifugation with 1 mL of DPBS, followed by DNA extraction. The extracted DNA was analyzed by agarose gel electrophoresis. A 0.8% agarose gel was prepared using 1× TEA solution. The samples and marker were stained with GelRed. After staining, a DNA marker (molecular weight of 250 bp to 10000 bp) was loaded into the leftmost lane, 100 ng of DNA extracted from non-cryopreserved cells was loaded into Lane 1, 200 ng of DNA extracted from non-cryopreserved cells was loaded into Lane 2, 100 ng of DNA extracted from cryopreserved cells was loaded into Lane 3, and 200 ng of DNA extracted from cryopreserved cells was loaded into Lane 4. Electrophoresis was performed at 140 V for 30 minutes. Agarose gel electrophoresis images were captured using a Gel Doc^{™} EZ gel imaging system.

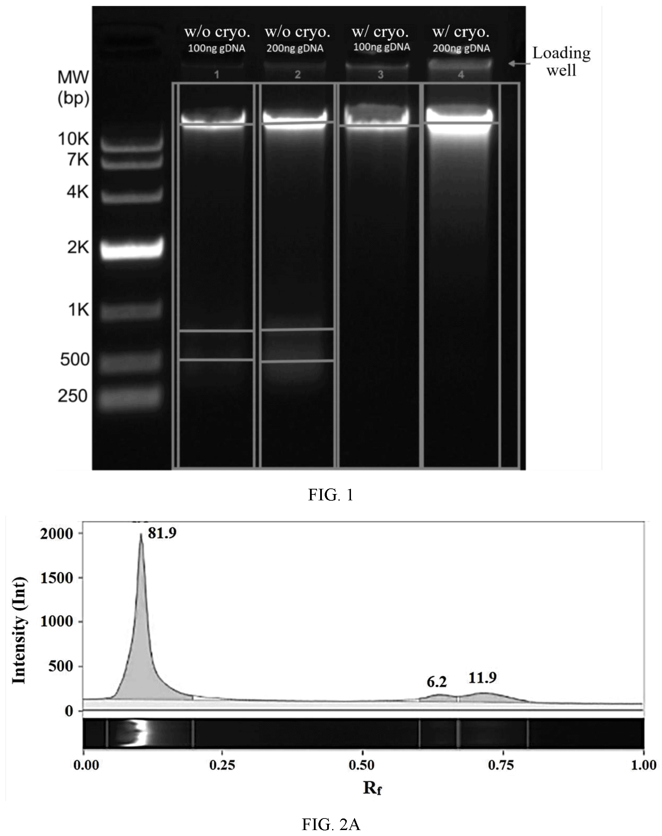

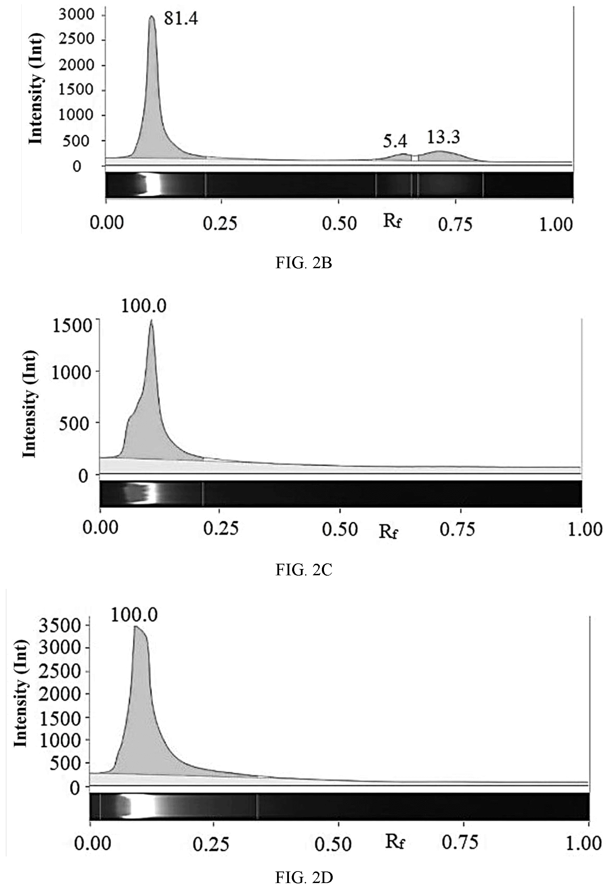

The agarose gel electrophoresis results are shown in FIG. 1, wherein the samples without cell cryopreservation (Lanes 1 and 2) exhibited three bands, which were >10 kb, between 500 bp and 1000 bp, and between 200 bp and 500 bp in size, respectively; whereas the samples subjected to cell cryopreservation (Lanes 3 and 4) exhibited only one band >10 kb in size. Image processing was performed using gel imaging software, and the results for Lanes 1 to 4 are shown in FIGs. 2A to 2D, respectively. FIGs. 2A and 2B showed that 80% of the DNA had a molecular weight greater than 10 kb, while approximately 20% of the DNA was distributed in small fragments of <1000 bp. FIGs. 2C and 2D showed that 100% of the DNA had a molecular weight greater than 10 kb. Nuclease treatment of non-cryopreserved cells is prone to causing degradation of genomic DNA and affecting the testing results.

## Claims

1. A method of nucleic acid extraction from engineered cells, comprising:
a) a pretreatment step, comprising a step of freezing and thawing the engineered cells, and a contact step of contacting the engineered cells with a nuclease;
b) a step of extracting intracellular nucleic acids.

2. The method according to claim 1, wherein the nuclease is an endonuclease, an exonuclease, or a combination thereof;
preferably, the nuclease is a deoxyribonuclease, a ribonuclease, or a universal nuclease;
more preferably, the nuclease is Benzonase or DnaseI.

3. The method according to claim 1 or 2, wherein the contact lasts for no more than 2 hours;
preferably, the contact lasts for 15 minutes to 60 minutes;
more preferably, the contact lasts for about 30 minutes.

4. The method according to any one of claims 1 to 3, wherein the density of the engineered cells during the contact is 1 × 10⁴ cells/mL to 1 × 10⁸ cells/mL;
preferably, the density of the engineered cells during the contact is 5 × 10⁵ cells/mL to 1 × 10⁷ cells/mL;
more preferably, the density of the engineered cells during the contact is 5 × 10⁵ cells/mL to 7.5 × 10⁶ cells/mL.

5. The method according to any one of claims 1 to 4, wherein the concentration of the nuclease during the contact is 50 to 2000 U/mL;
preferably, the concentration of the nuclease during the contact is 75 to 1500 U/mL;
more preferably, the concentration of the nuclease during the contact is 100 to 1000 U/mL.

6. The method according to any one of claims 1 to 5, wherein the ratio of the nuclease to the cells during the contact is 1 × 10⁴ to 2 × 10⁷ cells per 1000 U of the nuclease;
preferably, the ratio of the nuclease to the cells during the contact is 5 × 10⁵ to 1.5 × 10⁷ cells per 1000 U of the nuclease;
more preferably, the ratio of the nuclease to the cells during the contact is 1 × 10⁶ to 7.5 × 10⁶ cells per 1000 U of the nuclease.

7. The method according to any one of claims 1 to 6, wherein the temperature for freezing the engineered cells is no more than -20°C;
preferably, the temperature for freezing the engineered cells is about -80°C.

8. A method of nucleic acid extraction from engineered cells, comprising:
a) a pretreatment step, comprising contacting the engineered cells with a nuclease for 15 minutes to 60 minutes, wherein the density of the engineered cells during the contact is 5 × 10⁵ cells/mL to 1 × 10⁷ cells/mL, and the concentration of the nuclease is 75 to 1500 U/mL;
b) a step of extracting intracellular nucleic acids;
preferably, the method comprises:
a) a pretreatment step, comprising freezing and thawing the engineered cells, and contacting the thawed engineered cells with a universal nuclease for about 30 minutes, the density of the engineered cells during the contact is 5 × 10⁵ cells/mL to 7.5 × 10⁶ cells/mL, and the concentration of the nuclease is 100 to 1000 U/mL;
b) a step of extracting intracellular nucleic acids;
more preferably, the method comprises:
a) a pretreatment step, comprising freezing and thawing the engineered cells, and contacting the thawed cells with Benzonase for 30 minutes, the density of the engineered cells during the contact is 1 × 10⁶ cells/mL to 7.5 × 10⁶ cells/mL, and the concentration of the nuclease is 1000 U/mL;
b) a step of extracting intracellular nucleic acids.

9. The method according to any one of claims 1 to 8, wherein the engineered cells are engineered mammalian cells;
preferably, the engineered cells are engineered immune cells or engineered tumor cells;
more preferably, the engineered cells are engineered T cells or engineered NK cells.

10. The method according to any one of claims 1 to 9, wherein the engineered cells comprise an exogenous nucleic acid introduced via a viral vector, a non-viral vector, or a physical method;
preferably, the exogenous gene is introduced into the engineered cells via a lentiviral vector, retroviral vector, adenoviral vector, adeno-associated viral vector, cationic liposome, polymer nanoparticle, transposon, electroporation, or microinjection;
more preferably, the exogenous gene is introduced into the engineered cells via a lentiviral vector.
